(19) Europäisches Patentamt

European Patent Office

Office européen des brevets

(11) **EP 0 554 162 B1**

(12) **FASCICULE DE BREVET EUROPEEN**

(45) Date de publication et mention de la délivrance du brevet:
**21.10.1998 Bulletin 1998/43**

(51) Int Cl.⁶: **C12P 7/64**, C12M 1/02, C12M 1/04
// (C12P7/64;, C12R1:89)

(21) Numéro de dépôt: **93400185.0**

(22) Date de dépôt: **26.01.1993**

(54) **Procédé de production sélective de lipides poly-insaturés à partir d'une culture de micro-algues du type porphyridium cruentum**

Verfahren zur selektiven Herstellung von Poly-ungesättigte-Lipiden aus einer Porphyridium Cruentum Mikroalgen Fermentationsbrühe

Process for selectively producing poly-insaturated lipids starting from a porphyridium cruentum micro-algae fermentation broth

(84) Etats contractants désignés:
**CH DE ES GB IT LI**

(30) Priorité: **28.01.1992 FR 9200861**

(43) Date de publication de la demande:
**04.08.1993 Bulletin 1993/31**

(73) Titulaire: **THALLIA Pharmaceuticals S.A.**
**69130 Ecully (FR)**

(72) Inventeurs:
• **Thepenier, Catherine**
**F-04100 Manosque (FR)**
• **Gudin, Claude**
**F-13100 Aix en Provence (FR)**
• **Sarrobert, Bruno**
**F-04100 Manosque (FR)**

(74) Mandataire: **Pernez, Helga et al**
**Breese-Majerowicz,**
**3, avenue de l'Opéra**
**75001 Paris (FR)**

(56) Documents cités:
• **MIRCEN JOURNAL OF APPLIED MICROBIOLOGY AND BIOTECHNOLOGY vol. 4, no. 2, 1988, pages 231 - 237 Y.K. LEE ET AL. 'Effect of temperature, light intensity and dilution rate on the cellular composition of red alga Porphyridium cruentum in light limited chemostat cultures'**
• **JOURNAL OF PHYCOLOGY vol. 24, no. 3, Septembre 1988, pages 328 - 332 ZVI COHEN ET AL. 'Effect of environmental conditions on fatty acid composition of the red alga Porphyridium cruentum: correlation to growth rate'**

Remarques:
Le dossier contient des informations techniques présentées postérieurement au dépôt de la demande et ne figurant pas dans le présent fascicule.

## Description

L'invention concerne un procédé de production sélective de lipides poly-insaturés à partir d'une culture de micro-algues du type Porphyridium Cruentum.

Les micro-algues cultivées en milieu marin (concentration en NaCl supérieure à 0,2 M) sont souvent riches en acides gras poly-insaturés à longue chaîne. Ces acides gras présentent au moins 20 atomes de carbone et généralement plus de quatre insaturations. Ce sont par exemple l'acide arachidonique (C 20 : 4ω6), l'acide eicosapentaénoïque (C 20 : 5w3) et l'acide docosahexaénoïque (C 22 : 6ω3).

Ces acides gras ont des propriétés hypocholestérolémiantes et diminuent les risques d'athérogénèse. Ces acides gras sont actuellement utilisés en diététique et consommés sous forme d'huiles de poissons. En effet, les poissons sont riches en lipides poly-insaturés car ils les accumulent en consommant les micro-algues du plancton. Cependant, les huiles de poissons présentent deux inconvénients majeurs, à savoir la présence de cholestérol et une forte odeur désagréable. Il serait donc très intéressant de remplacer ces huiles de poissons directement par les huiles extraites des micro-algues, qui elles, ne contiennent pas de cholestérol et n'ont pas d'odeur.

En conséquence, on a cherché à cultiver des micro-algues.

Parmi les micro-algues, les rhodophycées telles que Porphyridium cruentum contiennent de façon majoritaire trois acides gras, un acide gras saturé prédominant (l'acide palmitique C 16 : 0) et deux acides gras poly-insaturés également prédominants (l'acide arachidonique (C 20 : 4) et l'acide eicosapentaénoïque (C 20 : 5). Environ 80% du C 20 : 5 se trouve dans les glycolipides présents dans les membranes du chloroplaste, tandis que 65% du C 20 : 4 se trouve dans les phospholipides majoritaires dans les membranes des cellules. Ces acides gras, dans des conditions favorables de production, représentent 2 à 3% de la matière sèche algale.

On sait déjà d'après l'article de Tim J. AHERN et al, "Arachidonic Acid Production by the Red Alga Porphyridium cruentum", Biotechnology and Bioengineering, vol. XXV, pp. 1057-1070 (1983) et l'article de Zvi COHEN et al., "Effect of Environmental Conditions on Fatty Acid Composition of the Red Alga Porphyridium cruentum : Correlation to Growth Rate", J. Phycol. 24, 328-332 (1988), que la teneur en acides gras poly-insaturés à l'intérieur de ces micro-algues varie fortement en fonction des conditions de culture et notamment des conditions de température. Dans ces techniques de l'art antérieur, les cultures de micro-algues sont toujours effectuées en une seule étape. Or, l'optimisation du profil en acides gras poly-insaturés se fait souvent à une température différente de celle de l'optimisation de la croissance des micro-algues. En conséquence, les cultures de micro-algues réalisées à des températures supérieures à 15°C donnent de faibles quantités d'acides gras et inversement, il n'est pas possible d'effectuer des cultures à une température inférieure à 15°C car alors, il n'y a plus de croissance et donc plus de production de biomasse.

L'invention a donc pour but d'optimiser la production de biomasse ainsi que la nature et la quantité de lipides riches en acides gras poly-insaturés.

A cet effet, l'invention concerne un procédé de production sélective de lipides poly-insaturés, à partir d'une culture de micro-algues du type Porphyridium cruentum.

Selon les caractéristiques de l'invention, ce procédé consiste à :

- cultiver dans un photobioréacteur tubulaire clôs la micro-algue Porphyridium cruentum, en suspension dans un milieu liquide de culture présentant une concentration en NaCl supérieure à 0,2 M et une température comprise entre 20 et 30°C environ,
- soutirer du photobioréacteur, au moins une partie de la biomasse obtenue et la placer dans une cuve pour l'enrichissement sélectif en lipides poly-insaturés,
- appliquer immédiatement à cette biomasse, pendant un temps donné, un écart thermique par rapport à la température de culture dans le photobioréacteur, de façon à modifier l'insaturation des acides gras des lipides.

Ce procédé permet ainsi d'optimiser d'abord la croissance des micro-algues en les cultivant dans des conditions optimales puis ensuite, séparément, lors de la deuxième étape du procédé, d'obtenir une production maximale de lipides poly-insaturés tout en choisissant la nature des acides gras contenus dans ces lipides.

Selon un premier mode de réalisation de l'invention, l'application de l'écart thermique à la biomasse consiste à amener celle-ci à une température comprise entre 4°C et 15°C environ, de façon à obtenir des lipides plus insaturés qu'ils ne l'étaient au départ dans les micro-algues.

En d'autres termes, alors que les micro-algues de type Porphyridium cruentum contenaient à l'origine environ 57% de C 20 : 4 et 43% de C 20 : 5, après le traitement par le froid, les micro-algues comprenaient entre 25 et 40% de C 20 : 4 et entre 60 et 75% de C 20 : 5.

Selon un second mode de réalisation de l'invention, l'application de l'écart thermique à la biomasse, consiste à amener celle-ci à une température comprise entre 30 et 40°C environ, de façon à obtenir des lipides moins insaturés qu'ils ne l'étaient au départ dans les micro-algues.

De façon avantageuse, on applique cet écart thermique pendant au moins 12 heures. Ainsi, après avoir favorisé la sélection de certains acides gras, on agit également sur la cinétique de la réaction.

De façon avantageuse, on introduit dans le milieu de culture de l'air enrichi en $CO_2$ à une concentration comprise entre 1 et 5% environ.

Ainsi, on favorise la photosynthèse et on améliore les conditions de culture des micro-algues à l'intérieur du photobioréacteur.

Enfin, le procédé de production selon l'invention consiste également à :

- concentrer les micro-algues enrichies en lipides poly-insaturés,
- broyer les micro-algues,
- séparer la phase liquide de la phase solide qui renferme la totalité des lipides poly-insaturés,
- extraire lesdits lipides par l'addition d'un solvant, et
- évaporer ledit solvant et récupérer les lipides poly-insaturés obtenus.

L'invention sera mieux comprise à la lecture de la description suivante d'un mode de réalisation préférentiel de l'invention donné à titre d'exemple illustratif et des dessins joints dans lesquels :

- la figure 1 représente un schéma d'ensemble d'un premier mode de réalisation d'une installation de production sélective de lipides poly-insaturés, utilisée pour la réalisation du procédé selon l'invention,
- la figure 2 représente un schéma d'ensemble d'un second mode de réalisation d'une installation de production sélective de lipides poly-insaturés utilisée pour la réalisation du procédé selon l'invention, et
- la figure 3 représente de façon plus détaillée l'un des éléments de la figure 1, à savoir un mode de réalisation de la cuve d'enrichissement sélectif en acides gras poly-insaturés.

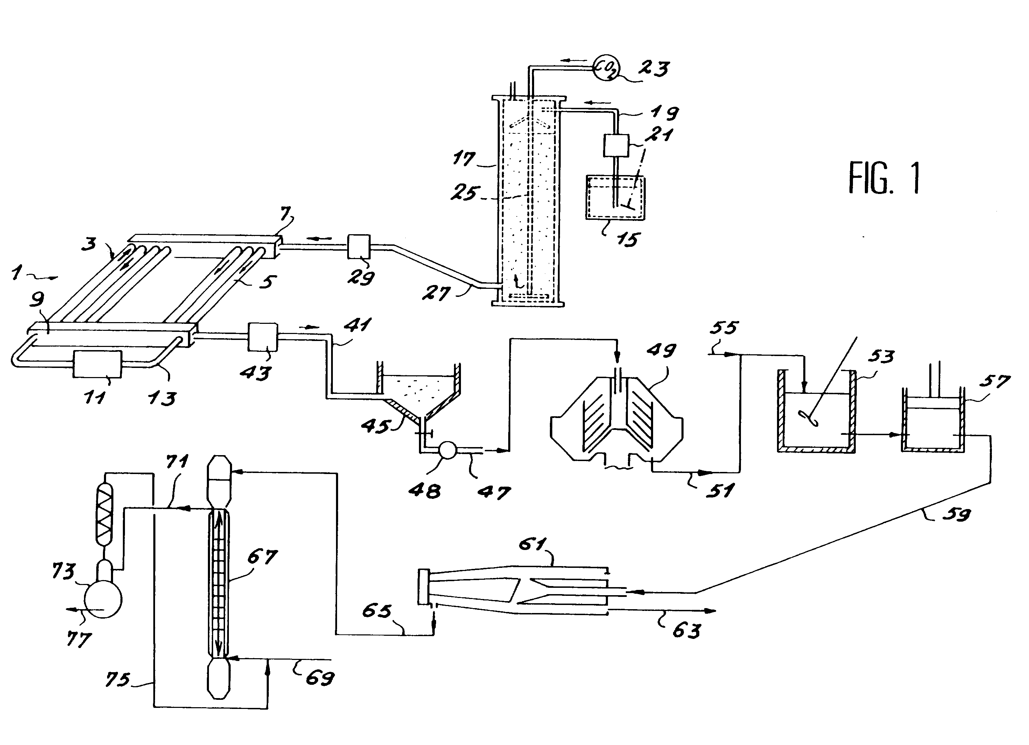

Comme illustré en figure 1, l'installation permettant de réaliser le procédé selon l'invention, comprend un photobioréacteur où sont cultivées les micro-algues du type <u>Porphyridium cruentum</u> et plusieurs appareils permettant d'extraire lesdits lipides de la biomasse obtenue.

La culture de micro-algues est effectuée dans un photobioréacteur 1, en mode continu ("chemostat"), ou ("turbidostat"), de façon à obtenir une qualité stable de biomasse. La photosynthèse a lieu dans ce photobioréacteur.

La photosynthèse est la transformation, grâce à l'énergie solaire, du dioxyde de carbone en matière première hydrocarbonée, l'oxygène étant le principal sous-produit de cette transformation biochimique. Cette réaction peut être symbolisée par l'équation de Myers suivante :

$$6,14\ CO_2 + 3,65\ H_2O + NH_3 \rightarrow C_{6,14}H_{10,3}O_{2,24}N + 6,85\ O_2$$

Le terme $C_{6,14}H_{10,3}O_{2,24}N$ correspond à la biomasse.

L'azote est apporté sous forme de nitrate, d'urée ou de sels d'ammonium introduits dans le milieu liquide contenant les micro-algues.

Un premier mode de réalisation du photobioréacteur permettant une culture optimale de micro-algues va maintenant être décrit.

Le photobioréacteur clos 1 comporte un récepteur solaire constitué par des tubes 3 parallèles, réalisés en matière plastique généralement transparente, telle que du polyéthylène, par exemple. A l'intérieur de ces tubes 3 circule le milieu de culture 5 contenant les micro-algues et les éléments nutritifs nécessaires à leur croissance.

De façon avantageuse, le milieu de culture 5 est constitué par de l'eau de mer artificielle du type milieu "Hémerick", présentant une concentration en NaCl supérieure à 0,2 M. Cette eau de mer est enrichie en oligo-éléments tels que le phosphate, le potassium ou l'azote. Son pH est compris entre 6 et 8 environ. Sa température est comprise entre 20 et 30°C. L'éclairement moyen est de 500 à 2000 kcal./$m^2$/j.

Des collecteurs 7 et 9 en polypropylène permettent de connecter entre eux les tubes 3 et d'assurer le passage du milieu de culture 5 d'un tube à l'autre.

Une pompe 11 montée sur une canalisation 13 met en communication l'entrée et la sortie du photobioréacteur 1. Cette pompe assure la circulation en continu du milieu de culture 5. Dans le mode de réalisation représenté en figure 1, le photobioréacteur fonctionne en continu. On pourrait également prévoir un second mode de réalisation, avec un fonctionnement discontinu.

Le milieu nourricier contenant les éléments nutritifs est préparé sous agitation dans une cuve 15 puis il est introduit au sommet d'un carbonateur 17 par une canalisation d'amenée 19 équipée d'une pompe 21 assurant le réglage du débit du milieu nourricier. Le gaz $CO_2$ est injecté sous pression depuis un réservoir 23 monté à l'entrée d'un tube plongeur 25 prévu à l'intérieur du carbonateur. Ce tube plongeur 25 permet l'alimentation en $CO_2$ à contre-courant du milieu nourricier arrivant par la canalisation 19. Le milieu nourricier carbonaté est injecté dans le photobioréacteur par une canalisation d'amenée 27 équipée d'une pompe 29. Le milieu de culture reçoit ainsi de l'air enrichi en $CO_2$ à une concentration comprise entre 1 et 5% environ.

Pour de plus amples détails sur la réalisation et le fonctionnement de ce capteur solaire (en continu ou en discontinu), on peut se référer au document FR-A-2 621 323.

La photosynthèse qui se déroule à l'intérieur du photobioréacteur 1 provoque un enrichissement de la culture en oxygène dissous, ce qui favorise la production d'anti-oxydants de type tocophérols.

A la sortie du photobioréacteur 1, une canalisation 41 équipée d'une pompe 43 permet l'acheminement en continu de la biomasse obtenue vers une cuve d'enrichissement en acides gras poly-insaturés 45.

A l'intérieur de cette cuve d'enrichissement 45, la biomasse subit un écart thermique. Une description détaillée de la structure de cette cuve et de son fonctionnement sera faite ultérieurement.

La biomasse enrichie en lipides poly-insaturés est prélevée de la cuve 45 par l'intermédiaire d'une canalisation 47 où elle est acheminée par une pompe 48 vers une centrifugeuse 49. Cette centrifugeuse permet de réaliser une étape de concentration où l'on sépare les corps cellulaires du milieu de culture. Cette étape de concentration peut également être réalisée par filtration ou par sédimentation.

Ensuite, la pâte de centrifugation est acheminée par une canalisation 51 vers une cuve d'homogénéisation 53 muni de moyens d'agitation, qui permettent d'obtenir une suspension de micro-algues homogène. Il est alimenté en milieu tamponné, c'est-à-dire en eau plus un tampon phosphate à pH 7,8, par une canalisation 55. La suspension de micro-algues est ensuite introduite dans un homogénéiseur broyeur 57. Ce broyeur 57 fonctionne par une alternance de pression et de dépression comprise entre 300 et 700, voire $1100.10^5$Pa. La température est contrôlée pour rester inférieure à 30°C de façon à ne pas altérer les lipides. Cette étape permet de faire éclater les micro-algues afin de séparer les fractions solides-liquides du contenu cellulaire.

La suspension de micro-algues broyées est ensuite acheminée par une canalisation 59 vers une décanteuse 61 permettant de séparer les morceaux de micro-algues, du milieu tamponné. Cette opération pourrait également être réalisée par centrifugation. Cette étape permet donc de séparer la phase liquide de la phase solide ou culot qui renferme tous les lipides poly-insaturés. La phase liquide est extraite en 63 et contient le milieu tamponné contenant entre autres des anti-oxydants tels que la vitamine C et la superoxyde dimutase, ainsi que des pigments tels que les phycobiliprotéines. Le culot solide est acheminé par une canalisation 65 vers une colonne d'extraction 67 fonctionnant à contre-courant.

Le résidu de micro-algues broyée est introduit par la partie haute de cette colonne d'extraction 67 tandis qu'un solvant est introduit à la base de la colonne en 69. Ce solvant est de préférence un solvant organique choisi par exemple parmi l'hexane ou le chloroforme. Ce solvant enrichi en acides gras poly-insaturés sort de la colonne dans sa partie supérieure en 71 et est envoyé à la base d'un évaporateur 73 fonctionnant aux environs de 40 à 50°C. Le solvant sortant de l'évaporateur 73, par une canalisation 75 est recyclé à la base de la colonne d'extraction 67.

On obtient à la sortie 77 de l'évaporateur, une huile enrichie en acides gras poly-insaturés, en agents anti-oxydants tels que les tocophérols ( $\alpha$, $\beta$, $\sqrt{}$ ) et les caroténoïdes (notamment le 1=carotène). La présence d'anti-oxydants dans l'extrait lipidique renfermant les acides gras, permet de protéger ces derniers contre les phénomènes d'oxydation. La teneur en anti-oxydant est fonction de la teneur en oxygène présente dans le milieu de culture du photobioréacteur (voir également la demande de brevet FR-A-2 656 874).

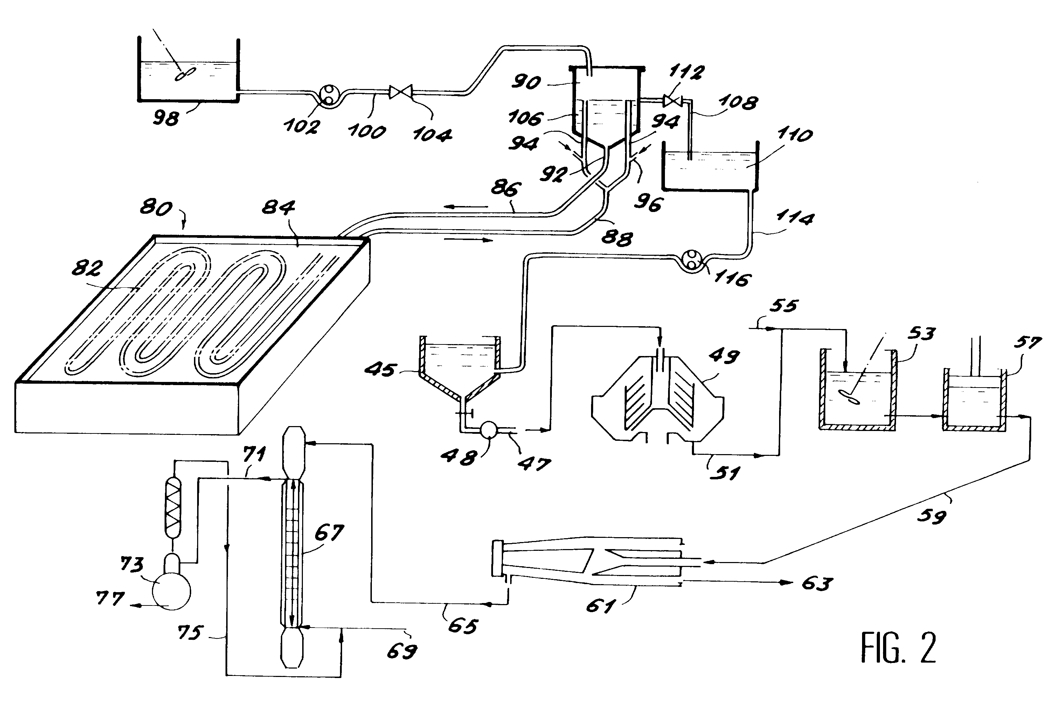

Un second mode de réalisation du photobioréacteur va maintenant être décrit en référence à la figure 2. Ce mode de réalisation est préférable au premier.

Le photobioréacteur 80 comprend un ensemble de tubes 82 transparents, réalisés généralement dans une matière plastique rigide telle que du Plexiglas (marque déposée). Ces tubes 82 sont reliés entre eux de façon à former un serpentin et sont disposés sur une étendue d'eau 84 thermostatée. Ce photobioréacteur 80 présente un tube d'entrée 86 et un tube de sortie 88.

Le photobioréacteur 80 est relié à un réservoir de charge 90, disposé en hauteur, à un niveau supérieur à celui dudit photobioréacteur. Le réservoir de charge 30 est relié au photobioréacteur 80 par une tubulure descendante 92 reliée au tube d'entrée 86 et par deux tubulures ascendantes 94 reliées au tube de sortie 88. Un injecteur 96 est placé à la base de chaque tubulure ascendante 94 et permet l'injection simultanée d'un gaz constitué d'air et de $CO_2$.

Le milieu nourricier contenant les éléments nutritifs est préparé sous agitation dans une cuve 98 puis il est introduit au sommet du réservoir de charge 90 par une canalisation d'amenée 100. Une pompe péristaltique 102 et une soupape 104 sont prévues sur cette canalisation d'amenée 100.

Le milieu de culture 106 (contenant les micro-algues et le milieu nourricier) est contenu dans le réservoir de charge 90, il s'écoule par gravité vers le photobioréacteur 80 par la tubulure descendante 92. Après avoir circulé dans le photobioréacteur 80, ce milieu de culture arrive dans le tube de sortie 88. Le milieu est alors remonté dans le réservoir de charge grâce à l'injection adéquate de gaz par l'injecteur 96, qui a pour effet de diminuer la densité du milieu liquide. Le gaz injecté permet non seulement de faire circuler le milieu de culture à l'intérieur du photobioréacteur 80 mais il permet en plus d'ajouter le $CO_2$ nécessaire à la photosynthèse.

Une canalisation 108 formant trop-plein permet d'évacuer en continu le milieu de culture provenant du réservoir de charge 90, en direction d'une cuve de stockage intermédiaire 110. La canalisation 108 présente une soupape 112. La cuve de stockage intermédiaire 110 est reliée à la cuve d'enrichissement 45 par une canalisation 114 sur laquelle est prévue une pompe péristaltique 116. Cette pompe 116 permet d'amener le milieu de culture vers la cuve d'enrichissement 45 à un débit donné qui peut être différent du débit de sortie du réservoir de charge 90, d'où la nécessité de la cuve de stockage intermédiaire 110.

Le reste du dispositif d'extraction est identique à ce qui a été décrit précédemment pour le premier mode de réalisation de la figure 1.

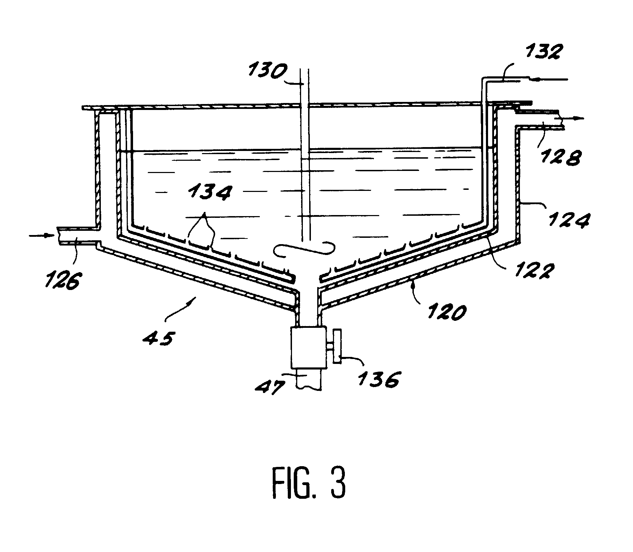

La structure de la cuve d'enrichissement 45 va maintenant être décrite plus en détail en faisant référence notamment à la figure 3. La cuve 45 est de préférence cylindrique et munie d'un fond conique 120. Elle est formée par une paroi double (paroi intérieure 122 et paroi extérieure 124).

Cette cuve est en outre munie d'un orifice d'entrée 126 et d'un orifice de sortie 128 permettant de faire circuler entre les deux parois 122 et 124 un liquide de refroidissement ou au contraire de réchauffement, permettant d'effectuer le choc thermique.

La forme de cette cuve est particulièrement bien adaptée pour obtenir une bonne régulation thermique et un bon écoulement des fluides. De plus, cette cuve est thermostatée afin de conserver la température chaude ou froide sélectionnée.

Cette cuve 45 comprend également un dispositif d'agitation 130 permettant d'assurer un brassage de la biomasse et de favoriser son contact avec la paroi intérieure 122 où se font les échanges thermiques. La cuve 45 comprend aussi un dispositif d'injection d'air 132 constitué par une canalisation s'étendant à l'intérieur de la cuve 45, le long de la paroi intérieure 122 et munie dans le fond de la cuve (partie conique) d'orifices 134 de sortie d'air. Ce dispositif d'injection d'air 132 permet d'éviter l'adhésion des micro-algues sur les parois et d'introduire éventuellement du $CO_2$ mélangé à de l'air.

Cette cuve comprend une vanne 136 prévue sur la canalisation de sortie 47. Cette vanne 136 permet de soutirer la culture afin d'effectuer les étapes ultérieures d'extraction du procédé. La biomasse peut être placée à un temps zéro dans la cuve 45 et soutirée dans sa totalité à la fin d'un temps donné (fonctionnement de type "batch" : discontinu) ou au contraire être renouvelée et soutirée en permanence (mode continu). Dans ce deuxième cas, la culture est apportée dans la cuve 45 en continu via une pompe dont on choisit le débit en fonction du temps de séjour souhaité dans la cuve, la culture est soutirée en continu par un trop-plein. On notera que sur cette figure 2 ni la pompe ni le trop-plein n'ont été représentés.

Enfin, cette cuve est généralement placée sous une intensité lumineuse comprise entre 20 et 300 $\mu E/m^2/s$.

La forme particulière de cette cuve 45 qui vient d'être décrite n'est toutefois qu'un mode particulier de réalisation. On pourrait réaliser une cuve avec une autre forme sans pour autant sortir du cadre de l'invention.

On a également pu montrer que la sélection préférentielle du C 20 : 4 et du C 20 : 5 dépendait du taux de dilution, c'est-à-dire de l'inverse du temps de séjour des micro-algues dans le photobioréacteur 1 ou 80.

Ce taux de dilution est généralement compris entre 1 et 0,1 $j^{-1}$ en fonction de l'intensité lumineuse reçue par les micro-algues, de leur photo-période, de la température, des conditions climatiques ou de l'acide gras visé. Les micro-algues cultivées avec un taux de dilution élevé, entre 0,3 et 1 $j^{-1}$ environ (temps de séjour court dans le photobioréacteur) contiennent plutôt plus de C 20 : 5 et inversement les micro-algues cultivées avec un taux de dilution faible, entre 0,1 et 0,3 $j^{-1}$ par exemple (temps de séjour long dans le photobioréacteur) contiennent plutôt plus de C 20 : 4. Ces valeurs sont toutefois données à titre indicatif.

Enfin, on notera que la production de biomasse augmente quand le taux de dilution augmente.

On a pu également constater qu'en fonction de l'amplitude de l'écart thermique appliqué et de la durée d'application de cet écart thermique, on modifiait la teneur de la biomasse en C 20 : 4 ou en C 20 : 5 et le rapport R entre C 20 : 5 et C 20 : 4. Pour obtenir principalement des C 20 : 5 (rapport R supérieur à 1), on effectue un écart thermique "froid", c'est-à-dire que l'on traite la biomasse à une température inférieure à 15°C et de préférence comprise entre 4°C et 15°C. Au contraire, si l'on souhaite obtenir une plus forte concentration de C 20 : 4 (rapport R inférieur à 1), on effectue un écart thermique "chaud", c'est-à-dire à une température supérieure à 30°C et de préférence comprise entre 30 et 40°C.

La durée d'application de cet écart thermique est fonction de la teneur en C 20 : 5 ou en C 20 : 4 souhaitée mais varie généralement entre 12 heures et 5 jours (120 heures).

Généralement, on combinera les taux de dilution et le sens des écarts thermiques pour faire varier le rapport R dans un sens ou dans l'autre.

Quatre exemples de cultures réalisées vont maintenant être décrits.

**Exemple 1**

La micro-algue <u>Porphyridium cruentum</u> a été cultivée dans un photobioréacteur clos, en mode continu, à une température comprise entre 22 et 25°C.

Le taux de dilution correspondant à l'inverse du temps de séjour dans le photobioréacteur était de 0,15 $^{j-l}$. En d'autres termes, ces micro-algues étaient cultivées pendant environ 6 jours dans le photobioréacteur. On a obtenu une productivité de biomasse de 10 g/m²/j. La répartition en acides gras majoritaires est la suivante :

- C 16 : 0 = 30,8% des acides gras totaux (AGT),
- C 20 : 4 = 23,4% des AGT,
- C 20 : 5 = 16,8% des AGT.

$$\text{Le rapport } R = \frac{C\ 20 : 5}{C\ 20 : 4} = 0,72$$

**Exemple 2**

On a cultivé des micro-algues de <u>Porphyridium cruentum</u> dans un photobioréacteur clos, en mode continu, à une température de 26°C, sous une forte intensité lumineuse.

On a fait varier le taux de dilution et la répartition en acide gras majoritaire était la suivante :

- Taux de dilution = 0,5 $^{j-1}$,

  - C 20 : 4 = 4% des acides gras totaux (AGT),
  - C 20 : 5 = 15% des AGT,
  - rapport R = 3,75,

- Taux de dilution = 0,16 j-l,

  - C 20 : 4 = 6% des AGT,
  - C 20 : 5 = 8% des AGT,
  - rapport R = 1,33.

Ceci montre que la quantité de C 20 : 5 croît quand le taux de dilution augmente.

**Exemple 3**

On a cultivé des micro-algues <u>Porphyridium cruentum</u> dans un photobioréacteur clos en mode continu à une température de 22°C et à un taux de dilution de 0,3 J$^{-1}$. Ensuite, on a appliqué un écart thermique en amenant la biomasse à une température de 12°C, sous une intensité lumineuse de 30 µE/m²/s et sans enrichissement en $CO_2$.

En fonction de la durée de séjour de la culture à 12°C (durée de l'écart thermique), on a observé les répartitions suivantes en acides gras majoritaires.

- Répartition initiale (culture provenant du photobioréacteur 1) :

    C 16 : 0 = 31,4% des acides gras totaux (AGT),
    C 20 : 4 = 27,1% des AGT,
    C 20 : 5 = 19,8% des AGT, et
    R = 0,73

Temps de séjour = 52 heures.
    C 16 : 0 = 26,9% des AGT,
    C 20 : 4 = 21,9% des AGT,
    C 20 : 5 = 30,6% des AGT,
    R = 1,40

Temps de séjour = 97 heures.
    C 16 : 0 = 28,3% des AGT,
    C 20 : 4 = 16,5% des AGT,
    C 20 : 5 = 33,4% des AGT,
    R = 2,02

Temps de séjour = 144 heures.
    C 16 : 0 = 27,5% des AGT,
    C 20 : 4 = 14,5% des AGT,
    C 20 : 5 = 38,1% des AGT,
    R = 2,63.

Lorsque l'on compare l'exemple 1 et l'exemple 2 avant la réalisation de l'écart thermique, on constate que malgré un taux de dilution légèrement différent, le rapport R est semblable, par contre avec un taux de dilution plus élevé (exemple 2), le pourcentage d'acides gras poly-insaturés, par rapport aux acides gras totaux, augmente.

Un écart thermique par le froid provoque une augmentation de la teneur en C 20 : 5 (et donc une augmentation de R). En effet, lorsque la micro-algue est soumise à un tel écart de température, elle réagit de façon à maintenir une certaine fluidité membranaire compatible avec la photosynthèse et les échanges membranaires.

## Exemple 4

On a cultivé des micro-algues de type <u>Porphyridium cruentum</u> à une température de 22°C et à un taux de dilution de 0,3 $j^{-l}$, dans un photobioréacteur tubulaire clos. On a ensuite appliqué un choc thermique en amenant la biomasse à une température de 12°C pendant 144 h. Ultérieurement, cette culture a été concentrée par centrifugation jusqu'à une teneur en matière sèche de 20 g/l. On a broyé cette culture dans un homogénéiseur à une pression de $500.10^5$Pa sous contrôle thermique puis on l'a centrifugé ou filtrée.

Les lipides poly-insaturés ont été extraits du culot solide par un mélange chloroforme :méthanol :eau (2:2:1,8). La fraction lipidique ainsi extraite représente 11,5% de la matière sèche algale. Cette fraction contient 36% d'acides gras totaux. Parmi ces acides gras, le C 20 : 4 en représente 14,5% et le C 20 : 5 représente 38,1%. L'huile ainsi obtenue, colorée par les caroténoïdes contient donc au total 5,2% de C 20 : 4 et 13,7% de 20 : 5 (R=2,63).

## Revendications

1. Procédé de production sélective de lipides poly-insaturés, à partir d'une culture de micro-algues du type <u>Porphyridium cruentum,</u> caractérisé en ce qu'il comprend les étapes suivantes consistant à :

   - cultiver dans un photobioréacteur (1, 80) tubulaire clos la micro-algue <u>Porphyridium cruentum,</u> en suspension dans un milieu liquide de culture (5, 106) présentant une concentration en NaCl supérieure à 0,2 M et une température comprise entre 20 et 30°C environ,
   - soutirer du photobioréacteur, au moins une partie de la biomasse obtenue et la placer dans une cuve (45) pour l'enrichissement sélectif en lipides poly-insaturés,
   - appliquer immédiatement à cette biomasse, pendant un temps donné, un écart thermique par rapport à la température de culture dans le photobioréacteur, de façon à modifier l'insaturation des acides gras des lipides.

2. Procédé de production sélective de lipides poly-insaturés selon la revendication 1, caractérisé en ce qu'il comprend les étapes complémentaires consistant à :

   - concentrer les micro-algues enrichies en lipides poly-insaturés,
   - broyer les micro-algues,
   - séparer la phase liquide de la phase solide qui renferme la totalité des lipides poly-insaturés,
   - extraire lesdits lipides par l'addition d'un solvant, et
   - évaporer ledit solvant et récupérer les lipides poly-insaturés obtenus.

3. Procédé de production sélective de lipides poly-insaturés selon la revendication 1, caractérisé en ce que l'application de l'écart thermique à la biomasse consiste à amener celle-ci à une température comprise entre 4°C et 15°C environ, de façon à obtenir des lipides plus insaturés qu'ils ne l'étaient au départ dans les micro-algues.

4. Procédé de production sélective de lipides poly-insaturés selon la revendication 1, caractérisé en ce que l'application de l'écart thermique à la biomasse consiste à amener celle-ci à une température comprise entre 30°C et 40°C environ, de façon à obtenir des lipides moins insaturés qu'ils ne l'étaient au départ dans les micro-algues.

5. Procédé de production sélective de lipides poly-insaturés selon la revendication 3 ou 4, caractérisé en ce que l'écart thermique est appliqué pendant une durée supérieure à 12 heures.

6. Procédé de production sélective de lipides poly-insaturés selon la revendication 5, caractérisé en ce que l'écart thermique est appliqué pendant une durée comprise entre 12 et 120 heures.

7. Procédé de production sélective de lipides poly-insaturés selon la revendication 1, caractérisé en ce que le taux de dilution de la culture est compris entre 0,1 et 1 $j^{-1}$.

**8.** Procédé de production sélective de lipides poly-insaturés selon la revendication 1, caractérisé en ce que l'on introduit dans le milieu de culture (5, 106) de l'air enrichi en $CO_2$ à une concentration comprise entre 1 et 5% environ.

**9.** Procédé de production sélective de lipides poly-insaturés selon la revendication 1, caractérisé en ce que le pH du milieu de culture (5, 106) dans le photobioréacteur (1) est compris entre 6 et 8 environ.

**10.** Procédé de production sélective de lipides poly-insaturés selon la revendication 1, caractérisé en ce qu'à l'intérieur du photobioréacteur (1, 80), l'éclairement moyen est compris entre 500 et 2000 $kcal/m^2/j$.

**11.** Procédé de production sélective de lipides poly-insaturés selon la revendication 2, caractérisé en ce que le broyage des micro-algues s'effectue dans un homogénéiseur broyeur (57) à une température inférieure à 30°C environ et à une pression supérieure à $300.10^5 Pa$.

**12.** Procédé de production sélective de lipides poly-insaturés selon la revendication 2, caractérisé en ce que le solvant utilisé pour l'extraction est un solvant organique choisi parmi le chloroforme ou l'hexane.

## Claims

**1.** Process for selective production of polyunsaturated lipids, from a culture of micro-algae of the <u>Porphyridium cruentum</u> type, characterised by the fact that it comprises the following steps consisting of:

- culturing the micro-alga <u>Porphyridium cruentum</u> in a closed tubular photobioreactor (1,80), in suspension in a liquid culture medium (5, 106) having a NaCl content greater than 0.2 M and a temperature of between approximately 20 and 30°C,

- drawing off from the photobioreactor at least a part of the biomass obtained and placing it in a vat (45) for selective enrichment in polyunsaturated lipids,

- applying immediately to this biomass, for a given period, a thermal variation relative to the culture temperature in the photobioreactor, so as to modify the unsaturation of the fatty acids of the lipids.

**2.** Process for selective production of polyunsaturated lipids as described in claim 1, characterised by the fact that it comprises the additional steps consisting of:

- concentrating the micro-algae enriched in polyunsaturated lipids,

- grinding the micro-algae,

- separating the liquid phase from the solid phase which contains the totality of the polyunsaturated lipids,

- extracting the said lipids by addition of a solvent, and

- evaporating the said solvent and recovering the polyunsaturated lipids obtained.

**3.** Process for selective production of polyunsaturated lipids as described in claim 1, characterised by the fact that the application of the thermal variation to the biomass consists of bringing the latter to a temperature of between approximately 4°C and 15°C, so as to obtain lipids more unsaturated than they were originally in the micro-algae.

**4.** Process for selective production of polyunsaturated lipids as described in claim 1, characterised by the fact that the application of the thermal variation to the biomass consists of bringing the latter to a temperature of between approximately 30°C and 40°C, so as to obtain lipids less unsaturated than they were originally in the micro-algae.

**5.** Process for selective production of polyunsaturated lipids as described in claim 3 or 4, characterised by the fact that the thermal variation is applied for a period greater than 12 hours.

**6.** Process for selective production of polyunsaturated lipids as described in claim 5, characterised by the fact that the thermal variation is applied for a period of between 12 and 120 hours.

**7.** Process for selective production of polyunsaturated lipids as described in claim 1, characterised by the fact that the rate of dilution of the culture is between 0.1 and $1^{d-1}$.

**8.** Process for selective production of polyunsaturated lipids as described in claim 1, characterised by the fact that air enriched with $CO_2$ at a concentration of between approximately 1 and 5% is introduced into the culture medium (5, 106).

**9.** Process for selective production of polyunsaturated lipids as described in claim 1, characterised by the fact that the pH of the culture medium (5, 106) in the photobioreactor (1) is between approximately 6 and 8.

**10.** Process for selective production of polyunsaturated lipids as described in claim 1, characterised by the fact that inside the photobioreactor (1, 80) the average illumination is between 500 and 2000 $kcal/m^2/d$.

**11.** Process for selective production of polyunsaturated lipids as described in claim 2, characterised by the fact that grinding of the micro-algae takes place in an homogeniser grinder (57) at a temperature of less than approximately 30°C and at a pressure greater than $300.10^5$ Pa.

**12.** Process for selective production of polyunsaturated lipids as described in claim 2, characterised by the fact that the solvent used for extraction is an organic solvent selected from chloroform or hexane.

## Patentansprüche

**1.** Verfahren zur selektiven Herstellung von ungesättigten Lipiden ausgehend von einer Mikroalgenkultur des Typs Porphyridium cruentum, gekennzeichnet durch die folgenden Schritte:

- die Mikroalge Porphyridium cruentum in einem zylindrischen geschlossenen Photobioreaktor (1, 80) in einem flüssigen Kulturmedium (5, 106) mit einer NaCl-Konzentration größer 0,2 M und bei einer Temperatur zwischen ca. 20 und 30°C kultivieren,
- wenigstens einen Teil der gewonnenen Biomasse aus dem Photobioreaktor herausnehmen und in einen Bottich (45) plazieren zur selektiven Anreicherung in mehrfach ungesättigten Lipiden,
- diese Biomasse sofort und während eines gegebenen Zeitraums einem Temperaturunterschied verglichen mit der Kulturtemperatur im Photobioreaktor unterziehen, um die Nicht-Sättigung der Fettsäuren der Lipide zu modifizieren.

**2.** Verfahren zur selektiven Herstellung von mehrfach ungesättigten Lipiden gemäß Anspruch 1, gekennzeichnet durch die folgenden ergänzenden Schritte:

- die in mehrfach ungesättigten Lipiden angereicherten Mikroalgen konzentrieren,
- die Mikroalgen zerkleinern,
- die flüssige Phase von der die gesamten mehrfach ungesättigten Lipide enthaltenden festen Phase trennen,
- die genannten Lipide durch Zuhilfenahme eines Lösungsmittels extrahieren und
- das genannte Lösungsmittel verdunsten und die gewonnenen mehrfach ungesättigten Lipide sammeln.

**3.** Verfahren zur selektiven Herstellung von mehrfach ungesättigten Lipiden gemäß Anspruch 1, dadurch gekennzeichnet, daß das Unterziehen der Biomasse einem thermischen Unterschied darin besteht, diese auf eine Temperatur zwischen ca. 4°C und 15°C zu bringen, um auf diese weise mehr ungesättigte Lipide zu erhalten als ursprünglich in den Mikroalgen enthalten.

**4.** Verfahren zur selektiven Herstellung von mehrfach ungesättigten Lipiden gemäß Anspruch 1, dadurch gekennzeichnet, daß das Unterziehen der Biomasse einem thermischen Unterschied darin besteht, diese auf eine Temperatur zwischen ca. 30°C und 40°C zu bringen, um auf diese Weise weniger ungesättigte Lipide zu erhalten als ursprünglich in den Mikroalgen enthalten.

**5.** Verfahren zur selektiven Herstellung von mehrfach ungesättigten Lipiden gemäß Anspruch 3 oder 4, dadurch gekennzeichnet, daß der thermische Unterschied während einer Dauer von mehr als 12 Stunden angebracht wird.

**6.** Verfahren zur selektiven Herstellung von mehrfach ungesättigten Lipiden gemäß Anspruch 5, dadurch gekennzeichnet, daß der thermische Unterschied während einer Dauer von 12 bis 120 Stunden angebracht wird.

**7.** Verfahren zur selektiven Herstellung von mehrfach ungesättigten Lipiden gemäß Anspruch 1, dadurch gekennzeichnet, daß die Verdünnungsrate der Kultur zwischen 0,1 und 1 $^{j-1}$ liegt.

**8.** Verfahren zur selektiven Herstellung von mehrfach ungesättigten Lipiden gemäß Anspruch 1, dadurch gekennzeichnet, daß man in das Kulturmedium (5, 106) mit $CO_2$ angereicherte Luft in einer Konzentration von ca. 1 bis 5% einführt.

**9.** Verfahren zur selektiven Herstellung von mehrfach ungesättigten Lipiden gemäß Anspruch 1, dadurch gekennzeichnet, daß der pH-Wert des Kulturmediums (5, 106) in dem Photobioreaktor (1) zwischen ca. 6 und 8 liegt.

**10.** Verfahren zur selektiven Herstellung von mehrfach ungesättigten Lipiden gemäß Anspruch 1, dadurch gekennzeichnet, daß innerhalb des Photobioreaktors (1, 80) die durchschnittliche Beleuchtung zwischen 400 und 2000 kcal/m$^2$/Tag liegt.

**11.** Verfahren zur selektiven Herstellung von mehrfach ungesättigten Lipiden gemäß Anspruch 2, dadurch gekennzeichnet, daß die Zerkleinerung der Mikroalgen in einem Apparat zum Homogenesieren und Zerkleinern (57) mit einer Temperatur kleiner als ca. 30°C und einem Druck höher als 300.10$^5$ Pa durchgeführt wird.

**12.** Verfahren zur selektiven Herstellung von mehrfach ungesättigten Lipiden gemäß Anspruch 2, dadurch gekennzeichnet, daß es sich bei dem für die Extrahierung verwendeten Lösungsmittel um ein organisches Lösungsmittel wie z.B. Chlorophorm oder Hexan handelt.

FIG. 1

FIG. 2

FIG. 3